# EUROPEAN PATENT SPECIFICATION

(11) **EP 3 666 252 B1**
(45) Date of publication and mention of the grant of the patent: **03.09.2025**
(21) Application number: 18843897.2
(22) Date of filing: 10.07.2018
(51) Int. Cl.: A61K 8/891, A61K 8/41, A61Q 5/12, A61Q 5/02

(54) **HAIR COSMETIC**
HAARKOSMETIKUM
PRODUIT COSMÉTIQUE POUR LES CHEVEUX

(30) Priority: 10.08.2017 JP 2017155875
(43) Date of publication of application: 17.06.2020
(73) Proprietor: KAO CORPORATION, Tokyo 103-8210 (JP)
(72) Inventor: TETSU, Makio, Tokyo 131-8501 (JP); AOYAGI, Kensuke, Tokyo 131-8501 (JP); YOKOTA, Yuuki, Tokyo 131-8501 (JP)
(74) Representative: Hoffmann Eitle
(86) International application number: PCT/JP2018/026081
(87) International publication number: WO 2019/031143

(56) References cited:
- EP-A2- 0 035 899
- WO-A1-2017/094625
- JP-A- 2000 344 633
- JP-A- 2006 265 205
- JP-A- 2016 121 091
- JP-A- 2017 019 748
- JP-A- S57 112 320
- JP-A- S62 126 111
- JP-A- S62 126 111
- SHIN-ETSU CHEMICAL CO: "Performance test results of silicone oil KF-96 of SHIN-ETSU Silicone", TECHNICAL MATERIALS, 1 May 2014 (2014-05-01), pages 1 - 36, XP055678280, Retrieved from the Internet <URL:http://www.silicone.jp/catalog/pdf/kf96_j.pdf> [retrieved on 20200320]
- SHIN-ETSU CHEMICAL CO: "Performance test results of silicone oil KF-96 of SHIN-ETSU Silicone", 1 May 2014 (2014-05-01), pages 1 - 36, XP055678280, Retrieved from the Internet <URL:http://www.silicone.jp/catalog/pdf/kf96_j.pdf> [retrieved on 20180910]

## Description

### Field of the Invention

The present invention relates to a hair cosmetic and a treatment method of hair.

### Background of the Invention

Since the moisture contained in the hair wetted with water after shampooing of hair is hardly removed, a lot of persons have a sense such that a drying behavior using a towel, a dryer, or the like is in general troublesome and time consuming. In recent years, requirements for desiring roominess and pleasantness in daily living are becoming strong due to diversification of a lifestyle, and persons who desire to finish simply the drying behavior after shampooing of hair are increasing.

Meanwhile, when the hair is allowed to stand without being thoroughly dried, arrangement of the hairstyle is impaired, thereby causing sleep-mussed hair or untidy hair.

With respect to the aforementioned requirements regarding the hair cosmetic, there are made the following proposals.

JP 2015-224200 A (PTL 1) and JP 2016-121091 A (PTL 2) disclose, as a hair cosmetic which after being applied to the hair, is capable of drying the hair for a short time, a hair cosmetic, such as a hair conditioner composition containing a specified higher alcohol, a cationic surfactant, and a dimethylpolysiloxane.

JP 1-168608 A (PTL 3) discloses a composition for hair drying, containing a volatile silicone, methylphenylpolysiloxane, and ethanol; and JP 2003-286137 A (PTL 4) discloses a cosmetic for hair quick drying, containing a lower alcohol and spherical powdery particles.

JP 2011-511830 A (PTL 5) discloses a hair treatment composition composed of an oil-in-water emulsion composition of a specified amino-modified dimethylpolysiloxane.

JP S62-126111A is directed to a hair cosmetic containing stably emulsified and dispersed silicone oil without lowering its lubrication effect, by combining a hydrophobic silicone oil to a di(long chain)-di(short chain) quaternary ammonium salt having a specific range of iodine value or degree of unsaturation.

EP 0 035 899 A2 describes a hair conditioning compositions in the form of an emulsion comprising a volatile conditioning agent selected from hydrocarbon and silicones, a nonionic, water soluble polymer, a cationic hair conditioning agent and water.

### Summary of the Invention

The present invention relates to [1] a hair cosmetic containing (A) a specified quaternary ammonium salt, (B) a linear dimethylpolysiloxane having a weight average molecular weight of 250 or more and 2,000 or less, and water, the hair cosmetic being used by applying on the hair and then rinsing away, wherein the content of the dimethylpolysiloxane (B) is 1 mass% or more and 60 mass% or less, a mass ratio of the component (B) to the component (A) [(B)/(A)] is 0.1 or more and 20 or less, and a receding contact angle (θ_{R}) of water droplet as measured by the expansion-contraction method, with respect to the surface of a polyethylene plate which is applied with the hair cosmetic and then subjected to water washing, is 82° or more and 110° or less; and [2] a treatment method of hair including (I) a step of applying the hair cosmetic on the hair and spreading it over the entire hair, and (II) a step of rinsing away the hair cosmetic with water from the entire hair.

### Detailed Description of the Invention

The above-cited PTLs 1 and 2 are concerned with a type of making the hair bundle wetted with water easy to come apart by a wind of a dryer, thereby shortening the drying time. However, there was room for improvement regarding an effect for time shortening.

In addition, all of the above-cited PTLs 3 to 5 are concerned with a hair treatment composition which is applied on the hair wetted with water and used without rinsing away and are neither a technology from the viewpoint of simplification of a behavior of from shampooing to drying nor shortening of the drying time of hair.

The present invention relates to a hair cosmetic to be used by applying on the shampooed hair and rinsing away, in which before starting a drying behavior of the hair, the moisture remaining among the hairs is naturally drained (dropped) by gravity as far as possible to shorten the drying time of the hair, thereby reducing a load of the drying behavior; and a treatment method of hair.

The present inventors have found that a hair cosmetic containing a specified quaternary ammonium salt and a dimethylpolysiloxane having a specified weight average molecular weight in specified amounts, wherein a receding contact angle (θ_{R}) of water droplet is controlled to 82° or more to 110° or less is able to solve the aforementioned problem.

Specifically, the present invention relates to the following [1] and [2].
[1] A hair cosmetic containing a quaternary ammonium salt (A) represented by the following general formula (1), a dimethylpolysiloxane (B) having a weight average molecular weight of 250 or more and 2,000 or less, and water, the hair cosmetic being used by applying on the hair and then rinsing away,
   wherein
   the component (B) is a linear dimethylpolysiloxane,
   the content of the dimethylpolysiloxane (B) is 1 mass% or more and 60 mass% or less,
   a mass ratio of the component (B) to the component (A) [(B)/(A)] is 0.1 or more and 20 or less, and
   a receding contact angle (θ_{R}) of water droplet as measured by the expansion-contraction method, with respect to the surface of a polyethylene plate which is applied with the hair cosmetic and then subjected to water washing, is 82° or more and 110° or less: 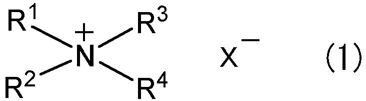
   wherein R¹ and R² each independently represent an alkyl group having 8 or more and 26 or less carbon atoms or an alkenyl group having 8 or more and 26 or less carbon atoms; R³ and R⁴ each independently represent an alkyl group having 1 or more and 3 or less carbon atoms; and X⁻ represents an anion.
[2] A treatment method of hair, including the following steps (I) and (II):
   (I) a step of applying the hair cosmetic as set forth in the above [1] on the hair and spreading it over the entire hair, and
   (II) a step of rinsing away the hair cosmetic with water from the entire hair.

In accordance with the present invention, it is possible to provide a hair cosmetic to be used by applying on the shampooed hair and then rinsing away, in which before starting a drying behavior of the hair, the moisture remaining among the hairs is naturally drained by gravity as far as possible to shorten the drying time of the hair, thereby reducing a load of the drying behavior; and a treatment method of hair.

### [Hair Cosmetic]

The hair cosmetic of the present invention is a hair cosmetic containing a quaternary ammonium salt (A) represented by the foregoing general formula (1), a linear dimethylpolysiloxane (B) having a weight average molecular weight of 250 or more and 2,000 or less, and water, the hair cosmetic being used by applying on the hair and then rinsing away,
wherein the content of the dimethylpolysiloxane (B) is 1 mass% or more and 60 mass% or less, a mass ratio of the component (B) to the component (A) [(B)/(A)] is 0.1 or more and 20 or less, and
a receding contact angle (θ_{R}) of water droplet as measured by the expansion-contraction method, with respect to the surface of a polyethylene plate which is applied with the hair cosmetic and then subjected to water washing, is 82° or more and 110° or less.

When the hair cosmetic of the present invention is used by applying on the shampooed hair and then rinsing away, before starting a drying behavior of the hair, by naturally draining the moisture remaining among the hairs by gravity as far as possible, it is possible to shorten the drying time of the hair and to reduce a load of the drying behavior. Though the reason for this is not elucidated yet, the following may be considered.

The low-molecular weight dimethylpolysiloxane (B) having a weight average molecular weight of 250 or more and 2,000 or less, which is used in the present invention, readily spreads on the hair surface at the time of applying on the hair because of its low viscosity, and moreover, its ability of repelling water is strong because of its low surface tension. Accordingly, it may be considered that the dimethylpolysiloxane (B) has such an effect for forming a hydrophobic film in water and naturally draining the moisture contained on the hair surface and among the hairs by gravity.

In addition, it may be considered that the quaternary ammonium salt (A) represented by the general formula (1) effectively strengthens the operation and effect of the aforementioned low-molecular weight dimethylpolysiloxane (B).

Then, by smoothening the hair cosmetic of the present invention over the hair and then rinsing away it, the aforementioned components (B) and (A) conjointly improve the receding contact angle (θ_{R}) of water droplet, whereby the moisture remaining on the hair surface and among the hairs after shampooing can be quickly dropped and drained by gravity. Accordingly, the moisture content to be naturally drained from the hair increases, whereby the moisture content remaining on the hair can be significantly reduced. As a result, it may be considered that the hair does not form a bundle but becomes easy to come apart, so that the hair is readily dried.

### <Receding Contact Angle (θ_{R}) of Water Droplet by Expansion-Contraction Method>

In the present invention, the receding contact angle (θ_{R}) of water droplet is determined by means of dynamic contact angle measurement by the expansion-contraction method. The receding contact angle (θ_{R}) of water droplet is an index exhibiting drainage properties of the wetted hair bundle.

The dynamic contact angle is known as a change in a dynamic contact angle supposing the state that a water droplet moves on the solid surface by washing, application, or the like. In general, the contact angle on the occasion when the interface of a water droplet advances is defined as an advancing contact angle (θₐ), and the contact angle on the occasion when it recedes is defined as a receding contact angle (θ_{R}). Details of this definition are described in Kenjiro Meguro and Kunio Esumi ed., Nure no Kiso to Oyo (The Elements and Applications of Wetting), (Realize Science & Engineering Center, published in 1989) and Toshio Ishii ed., Evaluation Technology for Interfacial Phenomena (published by Technosystem Co., Ltd., pp. 35-37 (2012)).

In the expansion-contraction method, a contact angle when on the occasion of piercing a tip of a needle of a syringe into a water droplet coming into contact with the solid surface and injecting a fixed amount of water, the water droplet advances is defined as the advancing contact angle (θₐ), and a contact angle when on the occasion of sucking water from a water droplet after injecting a fixed amount of water, the water droplet recedes is defined as the receding contact angle (θ_{R}).

The dynamic contact angle by the expansion-contraction method in the present invention can be, for example, measured at room temperature (25°C) by using an automatic contact angle meter (DropMaster), manufactured by Kyowa Interface Science Co., Ltd. Specifically, the receding contact angle (θ_{R}) can be determined by determining an average value of contact angles when ion exchange water is injected at a rate of 6.00 µL/s into a water droplet of ion exchange water in an initial amount of 50 µL for a fixed time and sucked, the measurement is performed at measurement intervals of 100 ms over 1,000 ms, and on the occasion of sucking water from the water droplet, the water droplet recedes. This value is a portion where after starting the suction of water, a fixed value appearing during a time of about 2,000 to 3,000 msec is exhibited.

More specifically, the measurement of the receding contact angle (θ_{R}) can be performed by the method described in the section of Examples.

In the present invention, the receding contact angle (θ_{R}) of water droplet as measured by the expansion-contraction method with respect to the surface of a polyethylene plate which is applied with the hair cosmetic and then subjected to water washing is 82° or more and 110° or less.

From the viewpoint of improving the drainage properties of the hair, this receding contact angle (θ_{R}) is preferably 83° or more, more preferably 85° or more, and still more preferably 87° or more, and it is preferably 108° or less, more preferably 105° or less, and still more preferably 100° or less.

The hair cosmetic having the aforementioned receding contact angle (θ_{R}) has the quaternary ammonium salt (A) represented by the general formula (1) and the dimethylpolysiloxane (B) having a weight average molecular weight of 100 or more and 3,000 or less and can be obtained by controlling the content of the component (B) to 0.3 mass% or more and 60 mass% or less in the hair cosmetic.

### <Quaternary Ammonium Salt (A) Represented by General Formula (1)>

The component (A) which is used in the present invention is a quaternary ammonium salt represented by the following general formula (1). As the component (A), specific compounds included in the general formula (1) can be used singly or combined as a mixture of two or more thereof.

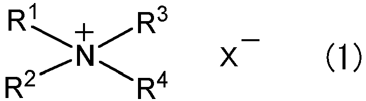

In the general formula (1), R¹ and R² each independently represent an alkyl group having 8 or more and 26 or less carbon atoms or an alkenyl group having 8 or more and 26 or less carbon atoms; R³ and R⁴ each independently represent an alkyl group having 1 or more and 3 or less carbon atoms; and X⁻ represents an anion.

As the alkyl group for R¹ and R², there is exemplified a linear or branched alkyl group having preferably 10 or more carbon atoms, and more preferably 12 or more carbon atoms, and preferably 22 or less carbon atoms, and more preferably 20 or less carbon atoms. Specific examples thereof include various octyl groups, various decyl groups, various dodecyl groups, various tetradecyl groups, various hexadecyl groups, various octadecyl groups, and various eicosyl groups.

As the alkenyl group for R¹ and R², from the viewpoint of strengthening the operation and effect of the component (B) and the viewpoint of shortening the drying time of the hair, there is exemplified a linear or branched alkenyl group having preferably 10 or more carbon atoms, and more preferably 12 or more carbon atoms, and preferably 22 or less carbon atoms, and more preferably 20 or less carbon atoms. Specific examples thereof include various octenyl groups, various decenyl groups, various dodecenyl groups, various tetradecenyl groups, various hexadecenyl groups, various octadecenyl groups, and various eicosenyl groups.

From the viewpoint of strengthening the operation and effect of the dimethylpolysiloxane (B), the alkyl group having 1 or more and 3 or less carbon atoms for R³ and R⁴ is a methyl group, an ethyl group, a propyl group, or an isopropyl group, preferably a methyl group or an ethyl group, and more preferably a methyl group.

From the viewpoint of costs and easiness of availability, the component (A) is preferably a quaternary ammonium salt represented by the general formula (2).

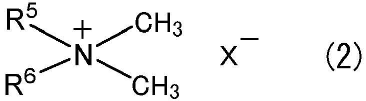

In the formula, R⁵ and R⁶ each independently represent a linear alkyl group having 10 or more and 22 or less carbon atoms; and X⁻ represents an anion.

In the general formula (2), R⁵ and R⁶ are each independently a linear alkyl group having preferably 12 or more carbon atoms, and preferably 20 or less carbon atoms, and more preferably 18 or less carbon atoms.

In the general formulae (1) and (2), examples of X⁻ that is an anion include organic or inorganic anions. Specific examples of the anion X⁻ include a halogen ion, an alkyl sulfate ion having 1 or more and 3 or less carbon atoms, an alkyl phosphate ion having 1 or more and 3 or less carbon atoms, a fatty acid ion having 12 or more and 18 or less carbon atoms, and a benzenesulfonate ion which may be substituted with 1 or more and 3 or less alkyl groups having 1 or more and 3 or less carbon atoms. Among those, a halogen ion and an alkyl sulfate ion having 1 or more and 3 or less carbon atoms are preferred; a chlorine atom, a bromine ion, a methyl sulfate ion, and an ethyl sulfate ion are more preferred; and a chlorine ion and a methyl sulfate ion are still more preferred.

Preferred examples of the quaternary ammonium salt represented by the general formula (1) or (2) include a didecyl(C 10) dimethyl ammonium salt, a dilauryl(C12) dimethyl ammonium salt, a dicoco dimethyl ammonium salt (C8 to 16), a dimyristyl(C14) dimethyl ammonium salt, a dicetyl(C16) dimethyl ammonium salt, a distearyl(C18) dimethyl ammonium salt, a diarachyl(C20) dimethyl ammonium salt, a dibehenyl(C22) dimethyl ammonium salt, a stearyl lauryl dimethyl ammonium salt, a dialkyl(C12 to 15) dimethyl ammonium salt, and a dialkyl(C12 to 18) dimethyl ammonium salt. Among those, from the viewpoint of strengthening the operation and effect of the component (B) and the viewpoint of shortening the drying time of the hair, a dialkyl(C12 to 15) dimethyl ammonium salt, a dicetyl(C16) dimethyl ammonium salt, and a dialkyl(C12 to 18) dimethyl ammonium salt are more preferred, and chlorides thereof are more preferred.

Examples of commercially available materials of the quaternary ammonium salt represented by the general formula (1) or (2) include "QUARTAMIN" Series, manufactured by Kao Corporation; VARISOFT 432PPG (dicetyldimonium chloride), manufactured by Evonik Nutrition & Care GmbH; RQUAD PC 2C-75 (dicocodimonium chloride), manufactured by Akzo Nobel N.V.; and LIPOQUAD 2C-75 (dicoconut alkyl dimethyl ammonium chloride), manufactured by Lion Specialty Chemicals Co., Ltd.

### <Dimethylpolysiloxane (B)>

The dimethylpolysiloxane (B) which is used in the present invention is a linear low-molecular weight dimethylpolysiloxane having a weight average molecular weight of 250 or more and 2,000 or less. This low-molecular weight dimethylpolysiloxane readily spreads on the hair surface because of its low viscosity, and its ability of repelling water is strong because of its low surface tension. Accordingly, the dimethylpolysiloxane (B) has such an effect for improving the natural drainage properties of the moisture remaining on the hair surface and among the hairs.

From the viewpoint of improving the natural drainage properties of the hair, the viewpoint of making the hair after drying with a towel easy to come apart, and the viewpoint of shortening the drying time of the hair, the weight average molecular weight of the dimethylpolysiloxane (B) is 250 or more, and it 2,000 or less, and preferably 1,500 or less.

In the case of using two or more dimethylpolysiloxanes having a different weight average molecular weight from each other, the weight average molecular weight of the dimethylpolysiloxane (B) means an arithmetic average molecular weight..

In the case where two or more dimethylpolysiloxanes are combined and used as the dimethylpolysiloxane (B) having an arithmetic average molecular weight of 250 or more and 2,000 or less, it is preferred that a dimethylpolysiloxane having a weight average molecular weight of 50,000 or more is not used because it hardly spreads on the hair surface at the time of applying on the hair, so that a uniform hydrophobic film is hardly formed in water, and there is a concern that the natural drainage effect of the hair is impaired.

From the viewpoint of improving the natural drainage properties of the hair, the viewpoint of making the hair after drying with a towel easy to come apart, and the viewpoint of shortening the drying time of the hair, the content of the dimethylpolysiloxane having a molecular weight of 100 or more and 2,000 or less in the dimethylpolysiloxane (B) is preferably 50 mass% or more, more preferably 55 mass% or more, still more preferably 70 mass% or more, and yet still more preferably 85 mass% or more, and it is preferably 100 mass% or less.

The weight average molecular weight of the dimethylpolysiloxane (B) and the amount corresponding to the molecular weight of 100 to 2,000 in the dimethylpolysiloxane (B) are a molecular weight expressed in terms of polystyrene as measured by the method described in the section of Examples.

In the present invention, a linear dimethylpolysiloxane is used as the dimethylpolysiloxane (B) from the viewpoint of improving the natural drainage properties of the hair, the viewpoint of making the hair after drying with a towel easy to come apart, and the viewpoint of shortening the drying time of the hair.

Specifically, from the viewpoint of improving the natural drainage properties of the hair, the viewpoint of making the hair after drying with a towel easy to come apart, and the viewpoint of shortening the drying time of the hair, a kinematic viscosity at 25°C of the linear or cyclic dimethylpolysiloxane is preferably 0.5 mm²/s or more, and more preferably 1 mm²/s or more, and it is preferably 25 mm²/s or less, more preferably 20 mm²/s or less, and still more preferably 10 mm²/s or less.

The measurement of the kinematic viscosity can be performed by the method described in the section of Examples.

Examples of commercially available materials of the linear dimethylpolysiloxane include KF-96 Series, manufactured by Shin-Etsu Chemical Co., Ltd.; SH200C Series and 2-1184 Fluid, manufactured by Dow Corning Toray Co., Ltd.; and Silsoft DML, Element 14 PDMS 5-JC, Element 14 PDMS 10-JC, and Element 14 PDMS 20-JC, manufactured by Momentive Performance Materials Inc.

Examples of the cyclic dimethylpolysiloxane include cyclopentasiloxane and cyclohexasiloxane. Examples of commercially available materials of the cyclic dimethylpolysiloxane include KF-995, manufactured by Shin-Etsu Chemical Co., Ltd.; SH245 Fluid, DC345 Fluid, and DC246 Fluid, manufactured by Dow Corning Toray Co., Ltd.; and TSF 405, SF 1258, and Silsoft 1217, manufactured by Momentive Performance Materials Inc.

### (Content of Each Component in Hair Cosmetic)

From the viewpoint of improving the emulsion dispersion stability of the dimethylpolysiloxane (B) and the viewpoints of allowing the dimethylpolysiloxane (B) to highly remain on the hair, improving the natural drainage properties of the moisture remaining on the hair surface and among the hairs, and enhancing the properties of the hair to come apart, the content of each of the components in the hair cosmetic is as follows.

The content of the component (A) in the hair cosmetic is preferably 0.2 mass% or more, more preferably 0.5 mass% or more, and still more preferably 1 mass% or more, and it is preferably 20 mass% or less, more preferably 15 mass% or less, still more preferably 10% or less, and yet still more preferably 6 mass% or less. Then, the content of the component (A) in the hair cosmetic is preferably 0.2 mass% or more or more and 20 mass% or less, more preferably 0.5 mass% or more and 15 mass% or less, still more preferably 1 mass% or more and 10 mass% or less, and yet still more preferably 1 mass% or more and 6 mass% or less.

The content of the component (B) in the hair cosmetic is 1 mass% or more, preferably 3 mass% or more, and more preferably 6 mass% or more, and it is 50 mass% or less, preferably 40 mass% or less, and more preferably 20 mass% or less. Then, the content of the component (B) in the hair cosmetic is 1 mass% or more and 50 mass% or less, preferably 3 mass% or more and 40 mass% or less, and more preferably 6 mass% or more and 20 mass% or less.

The content of water in the hair cosmetic is preferably 30 mass% or more, more preferably 40 mass% or more, still more preferably 50 mass% or more, and yet still more preferably 60 mass% or more, and it is preferably 97 mass% or less, more preferably 95 mass% or less, still more preferably 92 mass% or less, and yet still more preferably 90 mass% or less.

A mass ratio of the component (B) to the component (A) [(B)/(A)] in the hair cosmetic is 0.1 or more, preferably 0.5 or more, more preferably 1 or more, and still more preferably 2 or more from the viewpoint of allowing the dimethylpolysiloxane (B) to highly remain on the hair and effectively hydrophobizing the hair, and it is 20 or less, preferably 18 or less, more preferably 15 or less from the viewpoint of stably emulsifying the dimethylpolysiloxane (B) and allowing it to highly remain on the hair. Then, the mass ratio of the component (B) to the component (A) [(B)/(A)] in the hair cosmetic is 0.1 or more and 20 or less, preferably 0.5 or more and 18 or less, more preferably 1 or more and 15 or less, and still more preferably 2 or more and 15 or less.

### (Other Components in Hair Cosmetic and Their Contents)

From the viewpoint of improving the smoothness at the time of applying on the hair, the feeling at the time of rinsing, the stability, and so on, in the hair cosmetic of the present invention, an oil component, an aliphatic higher alcohol, a polyhydric alcohol, a silicone other than the dimethylpolysiloxane (B), various polymers exclusive of silicones and proteins, and so on can be further contained. Furthermore, arbitrary components, such as a solubilizing agent, a surfactant, a diluent, a touch improver, a hair repairing agent, a chelating agent, an antioxidant, a humectant, an ultraviolet absorber, a pH adjustor, and a perfume can be suitably added.

The oil component is preferably one that is liquid at room temperature. Specific examples thereof include hydrocarbon oils, such as an α-olefin oligomer, liquid isoparaffin, liquid paraffin, and squalane; triglycerides, such as glyceryl trioctanoate, avocado oil, olive oil, sesame oil, rice-bran oil, safflower oil, soybean oil, corn oil, rapeseed oil, castor oil, cottonseed oil, mink oil, glyceryl tri-2-ethylhexanoate, and caprylic/capric triglyceride; fatty acids, such as oleic acid and isostearic acid; and ester oils, such as isopropyl myristate, butyl myristate, isopropyl palmitate, ethyl oleate, ethyl linoleate, isopropyl linoleate, cetyl caprylate, hexyl laurate, decyl myristate, decyl oleate, oleyl oleate, isostearyl laurate, isotridecyl myristate, isocetyl myristate, isostearyl myristate, octyldodecyl myristate, octyl palmitate, isocetyl palmitate, isostearyl palmitate, propylene glycol dioleate, isodecyl oleate, isopropyl isostearate, cetyl 2-ethylhexanoate, stearyl 2-ethylhexanoate, propylene glycol dicaprylate, and propylene glycol dioleate.

There is a concern that the oil component weakens the effect of the dimethylpolysiloxane (B) and lowers the natural drainage properties of the hair, and therefore, its content is preferably 1 mass% or less, more preferably 0.5 mass% or less, and still more preferably 0.1 mass% or less in the hair cosmetic, and preferably, the oil component is not contained.

Examples of the aliphatic higher alcohol include alcohols having a linear or branched alkyl group or alkenyl group having preferably 8 or more and 26 or less carbon atoms, more preferably 12 or more and 22 or less carbon atoms, and still more preferably 16 or more and 20 or less carbon atoms. Specifically, examples thereof include myristyl alcohol, cetyl alcohol, stearyl alcohol, arachyl alcohol, behenyl alcohol, isostearyl alcohol, 2-octyl dodecanol, oleyl alcohol, and mixtures thereof.

There is a concern that the aliphatic higher alcohol weakens the effect of the component (B) and lowers the natural drainage properties of the hair, and therefore, its content is preferably 10 mass% or less, more preferably 5 mass% or less, still more preferably 3 mass% or less, and yet still more preferably 1 mass% or less in the hair cosmetic, and preferably, the aliphatic higher alcohol is not contained.

From the same viewpoint as that as mentioned above, a mass ratio of the aliphatic higher alcohol to the component (A) [(aliphatic higher alcohol)/(component (A))] is preferably 0.2 or less, more preferably 0.1 or less, and still more preferably 0.05 or less.

Examples of the polyhydric alcohol include ethylene glycol, diethylene glycol, a polyethylene glycol (number average molecular weight: less than 1,000), propylene glycol, dipropylene glycol, a polypropylene glycol (number average molecular weight: less than 1,000), glycerin, diglycerin, a polyglycerin, isoprene glycol, and 1,3-butylene glycol.

There is a concern that the polyhydric alcohol weakens the effect of the dimethylpolysiloxane (B) and lowers the natural drainage properties of the hair, and therefore, its content is preferably 5 mass% or less, more preferably 3 mass% or less, and still more preferably 1 mass% or less in the hair cosmetic, and preferably, the polyhydric alcohol is not contained.

Examples of the various polymers exclusive of silicones and proteins include natural polymers and low-molecular weight polymers resulting from hydrolysis thereof. Furthermore, there are exemplified (i) hydrophobized polymers obtained by subjecting the foregoing polymer to an addition reaction with a hydrophobic group of a fatty acid, an alcohol, or the like; (ii) hydrophilic polymers obtained by adding a hydrophilic group, such as a cation group, an anion group, and an ampholytic group, e.g., betaine, to the foregoing polymer; and (iii) amphipathic polymers obtaining by adding both a hydrophobic group and a hydrophilic group to the foregoing polymer.

Specifically, there are exemplified polysaccharides obtained by subjecting a fatty acid having 16 to 18 carbon atoms to ester bonding; polysaccharides obtained by subjecting a higher alcohol having 1 to 18 carbon atoms to ether bonding; cationized polysaccharides (e.g., cationized cellulose and cationized guar gum); and alkyl cationized polysaccharides (e.g., an alkyl cation-modified cellulose).

Examples of polymers obtained through polymerization of a synthesis system include polymers obtained by performing a reaction of a functional group having an unsaturated bonding group or a cyclic group and then performing polymerization. For example, there are exemplified polymers obtained through a reaction of acrylic acid or a derivative thereof, oxazoline or a derivative thereof, or the like.

There is a concern that such a polymer weakens the effect of the dimethylpolysiloxane (B) and lowers the natural drainage properties of the hair, and therefore, the content of each of the components is preferably 1 mass% or less, more preferably 0.5 mass% or less, still more preferably 0.1 mass% or less, and yet still more preferably 0.05 mass% or less in the hair cosmetic, and preferably, the respective component is not contained.

Examples of the silicone other than the dimethylpolysiloxane (B) include linear polysiloxanes having a weight average molecular weight of more than 3,000, such as dimethylpolysiloxane, methylphenylpolysiloxane, methylhydrogenpolysiloxane, and dimethiconol; modified silicones, such as polyether-modified silicones (e.g., polyoxyethylene (POE)-modified silicones and polyoxypropylene (POP)-modified silicones), polyamino-modified silicones, alcohol-modified silicones (e.g., glycerin-modified silicones), fatty acid-modified silicones, and fluorine-modified silicones; and silicon resins.

The content of the silicone other than the dimethylpolysiloxane (B) is preferably 1 mass% or less, more preferably 0.5 mass% or less, and still more preferably 0.1 mass% or less in the hair cosmetic, and preferably, the silicone other than the dimethylpolysiloxane (B) is not contained.

From the viewpoint of reducing any damage to the hair, or the like, the hair cosmetic of the present invention can contain one or more components selected from an amino acid, a peptide, a protein, an enzyme, gallic acid and a derivative thereof, sterol and a derivative thereof, a plant extract, and a dextrin fatty acid ester. There is a concern that such a component weakens the effect of the dimethylpolysiloxane (B) and lowers the natural drainage properties of the hair, and therefore, the content of each of the components is 1 mass% or less, more preferably 0.5 mass% or less, still more preferably 0.1 mass% or less, and yet still more preferably 0.05 mass% or less in the hair cosmetic, and preferably, the respective component is not contained.

From the viewpoint of enhancing the solubility and dispersibility of various blending components, such as an oil, a polymer, and a plant extract, the hair cosmetic of the present invention can further contain a surfactant other than the component (A). Specifically, the hair cosmetic of the present invention can contain (i) an anionic surfactant, (ii) an ampholytic surfactant, (iii) a nonionic surfactant, or (iv) a cationic surfactant other than the component (A) and the dialkyl cationic surfactant. There is a concern that such a component weakens the effect of the dimethylpolysiloxane (B) due to a relation with other components and lowers the natural drainage properties of the hair, and therefore, the content of each of the components is 1 mass% or less, more preferably 0.5 mass% or less, still more preferably 0.1 mass% or less, and yet still more preferably 0.05 mass% or less in the hair cosmetic, and preferably, the respective component is not contained.

### <Production of Hair Cosmetic>

The hair cosmetic of the present invention can be produced in the usual way. For example, purified water, such as ion exchange water, is heated at preferably 50°C or higher, and more preferably 55°C or higher, and preferably 95°C or lower, and more preferably 75°C or lower and mixed with the component (A) and the component (B), and the mixture is made uniform. Thereafter, if desired, an arbitrary component or components are further added and made uniform, followed by allowing the solution to stand for cooling. As the case may be, an acid or a base is added, and the pH is adjusted, whereby the hair cosmetic of the present invention can be obtained.

### (pH)

The pH of the hair cosmetic of the present invention is preferably 2 or more, more preferably 2.5 or more, and still more preferably 3 or more, and it is preferably 6.5 or less, more preferably 5.5 or less, and still more preferably 4.5 or less. The pH of the hair cosmetic is a value at 25°C when diluted 20 times by mass with water.

### (Form, etc. of Hair Cosmetic)

The formulation of the hair treatment agent of the present invention is not particularly limited, and may be an arbitrary formulation, such as a liquid, a foam, a paste, and a cream, can be taken, with the formulation being preferably a liquid, a paste, or a cream and more preferably a liquid.

As the form of the hair cosmetic of the present invention, there are preferably exemplified those to be used after shampooing within a bathroom, such as a hair rinse, a hair conditioner, a hair treatment, and a hair pack, namely those which after applying on the hair, are applied well smoothly over the hair and then rinsed away. Among those, from the viewpoint of exhibiting the effects of the present invention, it is more preferred to use the hair cosmetic of the present invention as a hair conditioner.

### [Treatment Method of Hair]

A treatment method of the hair of the present invention includes the following steps (I) and (II):
(I) a step of applying the hair cosmetic as set forth in the above [1] on the hair and spreading it over the entire hair, and
(II) a step of rinsing away the hair cosmetic with water from the entire hair.

In the step (I), the hair cosmetic of the present invention is first applied on the damp or wetted hair.

At this time, the hair cosmetic may be applied on the hair by hands, or the hair cosmetic may be applied on the hair by using a tool, such as a brush. Preferably, the hair cosmetic is applied on the shampooed hair.

Thereafter, the applied hair cosmetic is spread over the entire hair. In this case, in order to allow the hair cosmetic to penetrate into the interior or surface of the hair, it is preferred to rub into the hair using hands or a tool. Its time is preferably within 10 minutes, and more preferably 30 seconds or more and 5 minutes or less.

Subsequently, in the step (II), the hair cosmetic which has been spread over the entire hair is rinsed away with water. The temperature of the water to be used for rinsing away may be a temperature causing no load on the body, and it is preferably 15°C or higher and 50°C or lower, and more preferably 25°C or higher and 45°C or lower. The rinsing time is preferably 5 seconds or more and 3 minutes or less.

By the aforementioned steps (I) and (II), at the stage of performing wiping-off of the wetted hair after shampooing with a towel and before starting a usual drying behavior for drying with a dryer, the moisture remaining on the hair surface and among the hairs is quickly drained by a gravity, whereby the moisture content of the hair can be significantly reduced. Accordingly, the time of subsequent wiping-off of the hair with a towel and drying with a dryer can be shortened, whereby the drying behavior can be reduced.

### Examples

### (1) Measurement of Weight Average Molecular Weight (Mw) of Dimethylpolysiloxane

The weight average molecular weight (Mw) of the dimethylpolysiloxane was measured by means of gel permeation chromatography (GPC).

The Mw was calculated by using a column having two mixed gel columns (Shodex K-804L, manufactured by Showa Denko K.K.) connected with each other as a column, using chloroform (one for high-performance liquid chromatography, manufactured by Kanto Chemical Co., Inc.) as a mobile phase and a diluent solvent, using a differential refractive index (RI) detector as a detector, and using a monodisperse polystyrene having an already-known molecular weight as a standard substance.

The amount corresponding to the molecular weight of 100 to 2,000 in the dimethylpolysiloxane was determined from an integral molecular weight distribution curve.

### (2) Measurement of Kinematic Viscosity of Dimethylpolysiloxane

The kinematic viscosity of the dimethylpolysiloxane was measured at 25°C by using an Ubbelohde viscometer on the basis of JIS Z8803: "Methods for viscosity measurement of liquid".

### (3) Measurement of Receding Contact Angle (θ_{R})

### (i) Preparation of substrate for measurement:

A polyethylene plate (500 mm × 500 mm × 1 mm, a product number: 6-619-01, manufactured by AS ONE Corporation) was chopped in a size of about 2 cm in square to prepare a test piece; a 50-time diluted aqueous solution of an RBS-50 solution (manufactured by Sigma-Aldrich Co. LLC) was used as a washing liquid; and the test piece was dipped in the washing liquid and stirred, and then evenly flooded, followed by allowing to stand at 25°C overnight. Thereafter, the washing liquid was rinsed away with ion exchange water, and the resulting test piece was allowed to stand for drying under conditions at 25°C.

The above-obtained test piece was dipped in a hair cosmetic sample for 2 seconds and then taken out into air at 25°C, and the surface of the test piece was subjected to water washing, specifically, rinse-washing with ion exchange water for 2 seconds. After rinse-washing, the drained-off test piece was immediately measured as a substrate for measurement with respect to the receding contact angle.

### (ii) Measurement of receding contact angle (θ_{R}).

Using an automatic contact angle meter, DM-501Hi (manufactured by Kyowa Interface Science Co., Ltd.), the receding contact angle of a water droplet generated on the aforementioned substrate for measurement was measured at 25°C by the expansion-contraction method.

Ion exchange water was used for the measurement. The amount of the ion exchange water at the initial time was set to 50 µL, ion exchange water was injected into this liquid droplet and sucked, and the receding contact angle at the time of suction was measured. The amount of injection and suction of the water exchange was set to 6.00 µL/s, and after the injection time reached 4,700 msec, the suction was immediately started and performed for 4,700 msec.

The measurement of the receding contact angle was performed in the following manner. That is, after completion of the injection of water, the suction of the water droplet was started; at the stage when an end part (contact line) of the water droplet started to recede by the suction of the ion exchange water, the measurement was performed at measurement intervals of 100 ms over 1,000 msec or so; and on the occasion of sucking water from the water droplet, an average value of contact angles when the water droplet receded was determined, thereby determining the receding contact angle (θ_{R}). This value is a portion where after starting the suction of water, a constant value appearing during a time of about 2,000 to 3,000 msec is exhibited.

### Examples 1 to 9 and Comparative Examples 1 to 5 (Examples 1 and 5-7 are Reference Examples)

In a wide-mouthed standard bottle (PS-No. 6, manufactured by Tokyo Glass Kikai Co., Ltd.), all of respective raw materials (inclusive of water) as shown in Table 1 were added to make 35 g in a total amount, and the bottle was then stoppered. The contents were heated and mixed in a warm water bath at 65°C for about one hour. At the point of time when the raw materials were thoroughly mixed, the bottle was taken out from the warm water bath and shaken by hands until the mixture was uniformly emulsified and dispersed through visual inspection. Thereafter, the resultant was allowed to stand for cooling in an environment at 25°C, thereby obtaining a hair conditioner composition whose temperature became 25°C.

The obtained hair conditioner was used and measured for the receding contact angle (θ_{R}), and then evaluated for "easiness of hair to come apart" and "degree of natural drainage of hair (%)" in the following methods.

The results are shown in Table 1 along with the data before the treatment with the hair conditioner.

Details of the respective components in Table 1 are as follows.

### <Component (A)>

· Dialkyl(C12 to 18) dimonium chloride: QUARTAMIN D-2345P, manufactured by Kao Corporation
· Dicetyldimonium chloride: VARISOFT 432PPG, manufactured by Evonik Nutrition & Care GmbH

### <Component (B)>

*1 to 7 are a dimethylpolysiloxane (DMPS), manufactured by Shin-Etsu Chemical Co., Ltd.
*1: KF-96L (Mw: 199, kinematic viscosity: 0.65 mm²/s at 25°C)
*2: KF-96A (Mw: 260, kinematic viscosity: 1 mm²/s at 25°C)
*3: KF-96L (Mw: 392, kinematic viscosity: 2 mm²/s at 25°C)
*4: KF-96A (Mw: 1,290, kinematic viscosity: 10 mm²/s at 25°C)
*5: KF-96A (Mw: 2,230, kinematic viscosity: 20 mm²/s at 25°C)
*6: KF-96A (Mw: 5,130, kinematic viscosity: 50 mm²/s at 25°C)
*7: KF-96A (Mw: 8,870, kinematic viscosity: 100 mm²/s at 25°C)
*8: SH245 Fluid, cyclopentasiloxane manufactured by Dow Corning Toray Co., Ltd., Mw: 371, kinematic viscosity: 4 mm²/s at 25°C

### <Evaluation Methods>

### (1) Easiness of hair to come apart after drying with towel:

A hair bundle of an Asian straight hair with no chemical treatment history (hair length: about 20 cm, dry weight: 9 g) was washed with warm tap water by using a plain shampoo and then thoroughly rinsed away. 3 g of the sample liquid was applied on the hair bundle in a wetted state after shampooing and thoroughly applied smoothly over the entire hair. The applied sample liquid was then rinsed away with warm tap water for 5 seconds, and the hair bundle was then squeezed with fingers and drained off until water droplets did not drop. The treated hair bundle was placed on a pan balance, and the hair bundle was impregnated with tap water such that the content of water became 9 g that is equal to the dry weight (9 g) of the hair bundle (18 g in total).

On a double-folded paper towel of 40 cm × 16 cm in square (a product name: Ellieair^{®} Pro-Wipe Soft Towel White, manufactured by Daio Paper Corporation, dry weight: about 10 g) placed on an acrylic plate of 32 cm × 16 cm (thickness: 4 mm, weight: 293 g), the aforementioned hair bundle having been impregnated with water was laid such that the hair bundle was not bent; the same paper towel of 40 cm × 16 cm in square was placed thereon; the assembly was sandwiched by an acrylic plate of 32 cm × 16 cm (thickness: 4 mm, weight: 293 g); and a metallic weight (850 g) was then placed thereon, followed by performing towel pressing for 2 minutes.

Thereafter, the root of the water-absorbed hair bundle was held and swung three times at an amplitude of about 30°, and the fineness (easiness to come apart) of the hair bundle was graded with the following three scores by means of organoleptic evaluation by three expert panelists and evaluated in terms of a total score of the three persons (perfect score: 9).

Score 3: Any portion of the hair bundle considerably finely comes apart.

Score 2: Though fine bundles exist, thick bundles partially remain.

Score 1: Fine bundles do not substantially exist, and thick bundles remain in the majority.

When the total score is 6 or more, a significant difference in the "easiness of hair to come apart" can be recognized.

### (2) Degree of natural drainage of hair (%):

A hair bundle of an Asian straight hair with no chemical treatment history (hair length: about 20 cm, dry weight: 9 g) was washed with warm tap water by using a plain shampoo having the following blending formulation and then thoroughly rinsed away. 3 g of the hair cosmetic sample was applied on the hair bundle in a wetted state after shampooing and thoroughly applied smoothly over the entire hair. The applied sample liquid was then rinsed away with warm tap water for 5 seconds, and the hair bundle was then squeezed with fingers and drained off until water droplets did not drop. The treated hair bundle was placed on a pan balance, and the hair bundle was impregnated with tap water such that the content of water became 9 g that is equal to the dry weight (9 g) of the hair bundle (18 g in total). Thereafter, the hair bundle was suspended on the vertical line of the pan balance such that the hair ends were positioned at about 5 cm above the pan, and the weight (drainage amount) of the dripped water droplet was measured with a lapse of time.

The drainage amount after three minutes of suspension was weighed, and the degree of natural drainage of hair (%) was calculated according to the following equation. Degree of natural drainage of hair (%) = [(Drainage amount (g) after three minutes of suspension)/(Water content (g) of hair bundle before suspension)] × 100 [(Water content (g) of hair bundle before suspension) = (Dry weight (g) of hair)]

When the degree of natural drainage (%) is 52% or more, shortening of the drying time can be sufficiently recognized.

### [Blending Formulation of Plain Shampoo (pH: 7.0)]

| | |
|---|---|
| 25% Polyoxyethylene (2.5) lauryl ether sulfuric acid sodium | 62.0 mass% |
| Lauric acid diethanolamide: | 2.3 mass% |
| Disodium edetate: | 0.15 mass% |
| Sodium benzoate: | 0.5 mass% |
| Sodium chloride: | 0.8 mass% |
| 75% Phosphoric acid: | Proper amount |
| Perfume, methylparaben: | Minute amount |
| Purified water: | Balance |
| Total | 100 mass% |

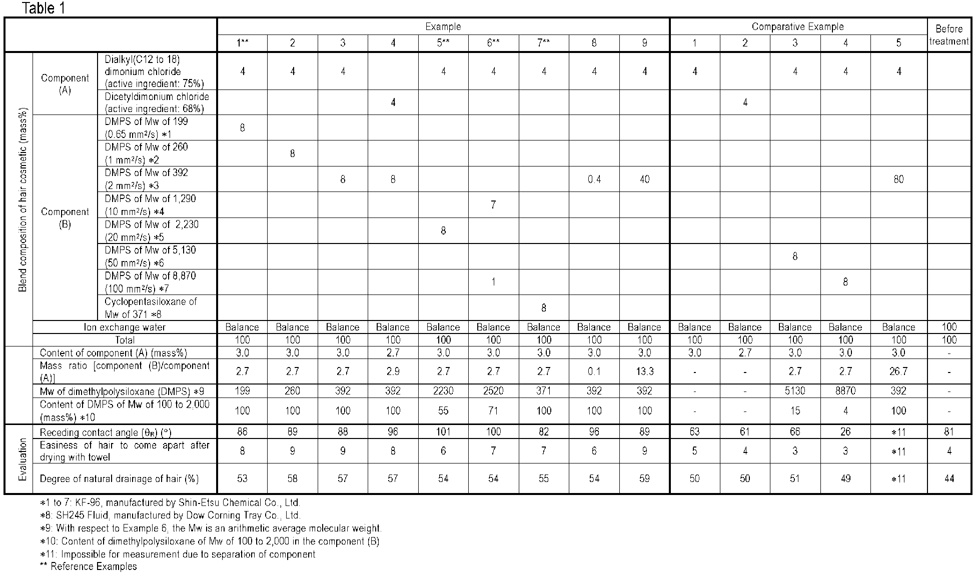

It is noted from Table 1 that the hair cosmetics of Examples 1 to 9 are large in the receding contact angle (θ_{R}) of water droplet and excellent in the easiness of the hair to come apart after drying with a towel and the degree of natural drainage of hair, as compared with the hair cosmetics of Comparative Examples 1 to 5.

### Industrial Applicability

In accordance with the hair cosmetic of the present invention, when used by applying on the shampooed hair and rinsing away, before starting a drying behavior of the hair, the moisture remaining among the hairs is naturally drained by gravity as far as possible, thereby making it possible to shorten the drying time of the hair and to reduce a load of the drying behavior.

## Claims

1. A hair cosmetic comprising a quaternary ammonium salt (A) represented by the following general formula (1), a dimethylpolysiloxane (B) having a weight average molecular weight of 250 or more and 2,000 or less, and water, the hair cosmetic being used by applying on the hair and then rinsing away,
wherein
the component (B) is a linear dimethylpolysiloxane,
the content of the dimethylpolysiloxane (B) is 1 mass% or more and 50 mass% or less,
a mass ratio of the component (B) to the component (A) [(B)/(A)] is 0.1 or more and 20 or less, and
a receding contact angle (θ_{R}) of water droplet as measured by the expansion-contraction method, with respect to the surface of a polyethylene plate which is applied with the hair cosmetic and then subjected to water washing, is 82° or more and 110° or less: 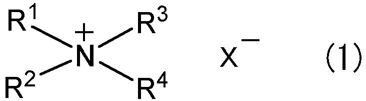
wherein R¹ and R² each independently represent an alkyl group having 8 or more and 26 or less carbon atoms or an alkenyl group having 8 or more and 26 or less carbon atoms; R³ and R⁴ each independently represent an alkyl group having 1 or more and 3 or less carbon atoms; and X⁻ represents an anion.

2. The hair cosmetic according to claim 1, wherein the component (A) is a quaternary ammonium salt represented by the following general formula (2): 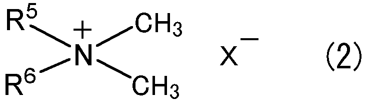 wherein R⁵ and R⁶ each independently represent a linear alkyl group having 10 or more and 22 or less carbon atoms; and X⁻ represents an anion.

3. The hair cosmetic according to claim 1 or 2, wherein the content of the component (A) is 0.2 mass% or more and 20 mass% or less.

4. The hair cosmetic according to any of claims 1 to 3, wherein the content of a dimethylpolysiloxane having a molecular weight of 100 or more and 2,000 or less in the component (B) is 50 mass% or more and 100 mass% or less.

5. The hair cosmetic according to any of claims 1 to 4, wherein the content of a silicone other than the dimethylpolysiloxane (B) is 1 mass% or less.

6. The hair cosmetic according to any of claims 1 to 5, wherein the content of an oil component is 0.1 mass% or less.

7. The hair cosmetic according to any of claims 1 to 6, wherein the content of an aliphatic higher alcohol is 1 mass% or less.

8. The hair cosmetic according to any of claims 1 to 7, wherein the content of a polyhydric alcohol is 1 mass% or less.

9. The hair cosmetic according to any of claims 1 to 8, wherein the content of a surfactant other than the component (A) is 0.5 mass% or less.

10. The hair cosmetic according to any of claims 1 to 9, which is used for a hair conditioner.

11. A treatment method of hair, comprising the following steps (I) and (II):
(I) a step of applying the hair cosmetic according to any one of claims 1 to 9 on the hair and spreading it over the entire hair, and
(II) a step of rinsing away the hair cosmetic with water from the entire hair.

12. Use of the hair cosmetic according to any of claims 1 to 9 as a hair conditioner.

## Patentansprüche

1. Haarkosmetikum, umfassend ein quaternäres Ammoniumsalz (A), dargestellt durch die folgende allgemeine Formel (1), ein Dimethylpolysiloxan (B) mit einem gewichtsmittleren Molekulargewicht von 250 oder mehr und 2.000 oder weniger, und Wasser, wobei das Haarkosmetikum durch Auftragen auf das Haar und anschließendes Ausspülen verwendet wird,
wobei
die Komponente (B) ein lineares Dimethylpolysiloxan ist,
der Gehalt des Dimethylpolysiloxans (B) 1 Massenprozent oder mehr und 50 Massenprozent oder weniger beträgt,
ein Massenverhältnis der Komponente (B) zu der Komponente (A) [(B)/(A)] 0,1 oder mehr und 20 oder weniger ist, und
ein Rückzugskontaktwinkel (receding contact angle) (θ_{R}) des Wassertropfens, gemessen durch die Expansions-Kontraktions-Methode, in Bezug auf die Oberfläche einer Polyethylenplatte, die mit dem Haarkosmetikum aufgetragen und dann einer Wasserwäsche unterzogen wird, 82° oder mehr und 110° oder weniger ist: 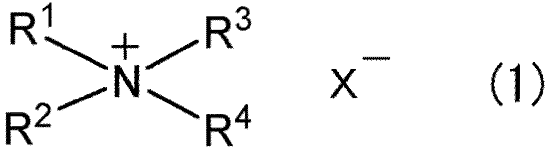
worin R¹ und R² jeweils unabhängig voneinander eine Alkylgruppe mit 8 oder mehr und 26 oder weniger Kohlenstoffatomen oder eine Alkenylgruppe mit 8 oder mehr und 26 oder weniger Kohlenstoffatomen darstellen; R³ und R⁴ jeweils unabhängig voneinander eine Alkylgruppe mit 1 oder mehr und 3 oder weniger Kohlenstoffatomen darstellen; und X⁻ ein Anion darstellt.

2. Haarkosmetikum gemäß Anspruch 1, wobei die Komponente (A) ein quaternäres Ammoniumsalz ist, das durch die folgende allgemeine Formel (2) dargestellt wird: 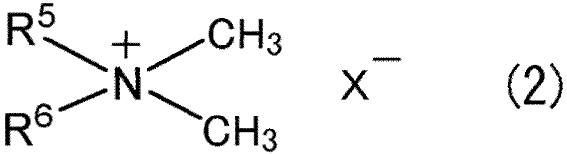 worin R⁵ und R⁶ jeweils unabhängig voneinander eine lineare Alkylgruppe mit 10 oder mehr und 22 oder weniger Kohlenstoffatomen darstellen; und X⁻ ein Anion darstellt.

3. Haarkosmetikum gemäß Anspruch 1 oder 2, wobei der Gehalt der Komponente (A) 0,2 Massenprozent oder mehr und 20 Massenprozent oder weniger beträgt.

4. Haarkosmetikum gemäß mindestens einem der Ansprüche 1 bis 3, wobei der Gehalt an einem Dimethylpolysiloxan mit einem Molekulargewicht von 100 oder mehr und 2.000 oder weniger in der Komponente (B) 50 Massenprozent oder mehr und 100 Massenprozent oder weniger beträgt.

5. Haarkosmetikum gemäß mindestens einem der Ansprüche 1 bis 4, wobei der Gehalt an einem anderen Silikon als dem Dimethylpolysiloxan (B) 1 Massenprozent oder weniger beträgt.

6. Haarkosmetikum gemäß mindestens einem der Ansprüche 1 bis 5, wobei der Gehalt an einer Ölkomponente 0,1 Massenprozent oder weniger beträgt.

7. Haarkosmetikum gemäß mindestens einem der Ansprüche 1 bis 6, wobei der Gehalt an einem aliphatischen höheren Alkohol 1 Massenprozent oder weniger beträgt.

8. Haarkosmetikum gemäß mindestens einem der Ansprüche 1 bis 7, wobei der Gehalt an einem Polyhydroxyalkohol (polyhydric alcohol) 1 Massenprozent oder weniger beträgt.

9. Haarkosmetikum gemäß mindestens einem der Ansprüche 1 bis 8, wobei der Gehalt an einem anderen Tensid als der Komponente (A) 0,5 Massenprozent oder weniger beträgt.

10. Haarkosmetikum gemäß mindestens einem der Ansprüche 1 bis 9, das für eine Haarspülung verwendet wird.

11. Verfahren zur Behandlung von Haaren, umfassend die folgenden Schritte (I) und (II):
(I) einen Schritt des Auftragens des Haarkosmetikums gemäß mindestens einem der Ansprüche 1 bis 9 auf das Haar und des Verteilens auf dem gesamten Haar, und
(II) einen Schritt des Ausspülens des Haarkosmetikums mit Wasser von dem gesamten Haar.

12. Verwendung des Haarkosmetikums gemäß mindestens einem der Ansprüche 1 bis 9 als Haarspülung.

## Revendications

1. Produit cosmétique pour les cheveux comprenant un sel d'ammonium quaternaire (A) représenté par la formule générale (1) suivante, un diméthylpolysiloxane (B) présentant un poids moléculaire moyen en poids de 250 ou plus et 2 000 ou moins, et de l'eau, le produit cosmétique pour les cheveux étant utilisé par application sur les cheveux et ensuite par rinçage,
dans lequel
le composant (B) est un diméthylpolysiloxane linéaire,
la teneur du diméthylpolysiloxane (B) est 1 % en masse ou plus et 50 % en masse ou moins,
le rapport de masse du composant (B) au composant (A) [(B)/(A)] est 0,1 ou plus et 20 ou moins, et
un angle de contact en retrait (Θ_{R}) d'une gouttelette d'eau tel que mesuré par la méthode d'expansion-contraction, par rapport à la surface d'une plaque de polyéthylène qui est appliquée avec le produit cosmétique pour les cheveux et ensuite soumise à un lavage à l'eau, est 82° ou plus et 110° ou moins : 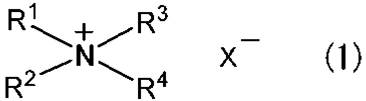
dans lequel R¹ et R² représentent chacun indépendamment un groupe alkyle possédant 8 atomes de carbone ou plus et 26 atomes de carbone ou moins ou un groupe alcényle possédant 8 atomes de carbone ou plus et 26 atomes de carbone ou moins ; R³ et R⁴ représentent chacun indépendamment un groupe alkyle possédant 1 atome de carbone ou plus et 3 atomes de carbone ou moins ; et X représente un anion.

2. Produit cosmétique pour les cheveux selon la revendication 1, dans lequel le composant (A) est un sel d'ammonium quaternaire représenté par la formule générale (2) suivante : 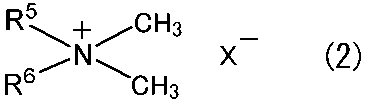 dans lequel R⁵ et R⁶ représentent chacun indépendamment un groupe alkyle linéaire possédant 10 atomes de carbone ou plus et 22 atomes de carbone ou moins ; et X représente un anion.

3. Produit cosmétique pour les cheveux selon la revendication 1 ou 2, dans lequel la teneur du composant (A) est 0,2 % en masse ou plus et 20 % en masse ou moins.

4. Produit cosmétique pour les cheveux selon l'une quelconque des revendications 1 à 3, dans lequel la teneur d'un diméthylpolysiloxane présentant un poids moléculaire de 100 ou plus et 2 000 ou moins dans le composant (B) est 50 % en masse ou plus et 100 % en masse ou moins.

5. Produit cosmétique pour les cheveux selon l'une quelconque des revendications 1 à 4, dans lequel la teneur d'un silicone autre que le diméthylpolysiloxane (B) est 1 % en masse ou moins.

6. Produit cosmétique pour les cheveux selon l'une quelconque des revendications 1 à 5, dans lequel la teneur d'un composant huileux est 0,1 % en masse ou moins.

7. Produit cosmétique pour les cheveux selon l'une quelconque des revendications 1 à 6, dans lequel la teneur d'un alcool supérieur aliphatique est 0,1 % en masse ou moins.

8. Produit cosmétique pour les cheveux selon l'une quelconque des revendications 1 à 7, dans lequel la teneur d'un alcool polyhydrique est 1 % en masse ou moins.

9. Produit cosmétique pour les cheveux selon l'une quelconque des revendications 1 à 8, dans lequel la teneur d'un tensioactif autre que le composant (A) est 0,5 % en masse ou moins.

10. Produit cosmétique pour les cheveux selon l'une quelconque des revendications 1 à 9, qui est utilisé pour un conditionneur capillaire.

11. Procédé de traitement de cheveux, comprenant les étapes (I) et (II) suivantes :
(I) une étape consistant à appliquer le produit cosmétique pour les cheveux selon l'une quelconque des revendications 1 à 9 sur les cheveux et à l'étaler sur l'ensemble des cheveux, et
(II) une étape consistant à rincer le produit cosmétique pour les cheveux avec de l'eau de l'ensemble des cheveux.

12. Utilisation du produit cosmétique pour les cheveux selon l'une quelconque des revendications 1 à 9 en tant qu'un conditionneur capillaire.
